# EUROPEAN PATENT APPLICATION

(11) **EP 4 775 957 A1**
(43) Date of publication of application: **15.07.2026**
(21) Application number: 26150346.0
(22) Date of filing: 06.01.2026
(51) Int. Cl.: G01N 21/03

(54) **COMPACT MULTI-PASS ABSORPTION GAS CELL**

(30) Priority: 12.01.2025 US 202563744285 P; 11.12.2025 US 202519416518
(71) Applicant: Honeywell International Inc., Charlotte, NC 28202 (US)
(72) Inventor: FENG, Chen, Charlotte, 28202 (US); LEE, Seong Eyl, Charlotte, 28202 (US); SHAFAI, Moin S, Charlotte, 28202 (US)
(74) Representative: Haseltine Lake Kempner LLP

(57) **Abstract**

The present disclosure provides a compact multi-pass absorption gas cell comprising a rectangular housing defining a chamber for receiving a sample to be analyzed, a light inlet positioned at a first end of the rectangular housing for introducing light beam into the chamber, a light outlet position at a second end of the rectangular housing, a first mirror component positioned at the first end comprising a first flat mirror and a first concave mirror array, and a second mirror component positioned at the second end comprising a second flat mirror and a second concave mirror array, wherein the light beam reflects multiple times between the first mirror component and the second mirror component and is refocused by the first concave mirror array and the second concave mirror array to achieve an optical path length for low absorption gas detection.

## Description

### CROSS-REFERENCE TO RELATED APPLICATIONS

This application claims priority to U.S. Provisional Patent Application No. 63/744,285, filed on January 12, 2025, which is incorporated herein by reference in its entirety.

### TECHNICAL FIELD

Embodiments of the present disclosure generally relate to gas cells, and, more particularly, to compact multi-pass absorption gas cells.

### BACKGROUND

Applicant has identified many technical challenges associated with multi-pass absorption gas cells. Through applied effort, ingenuity, and innovation, many of these identified problems have been solved by developing solutions that are included in embodiments of the present disclosure, many examples of which are described in detail herein.

### SUMMARY

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

According to an aspect of the present disclosure, a compact multi-pass absorption gas cell is provided. The compact multi-pass absorption gas cell comprises a rectangular housing defining a chamber for receiving a sample to be analyzed. The compact multi-pass absorption gas cell comprises a light inlet positioned at a first end of the rectangular housing for introducing light beam into the chamber. The compact multi-pass absorption gas cell comprises a light outlet position at a second end of the rectangular housing. The compact multi-pass absorption gas cell comprises a first mirror component positioned at the first end, the first mirror component comprising a first flat mirror and a first concave mirror array. The compact multi-pass absorption gas cell comprises a second mirror component positioned at the second end, the second mirror component comprising a second flat mirror and a second concave mirror array, wherein the light beam reflects multiple times between the first mirror component and the second mirror component and is refocused by the first concave mirror array and the second concave mirror array to achieve an optical path length for low absorption gas detection.

According to other aspects of the present disclosure, the compact multi-pass absorption gas cell may include one or more of the following features. The first concave mirror array and the second concave mirror array may each comprise a plurality of concave mirrors. The light beam may reflect between the first mirror component and the second mirror component in a rectangular row-by-row pattern. The light inlet and light outlet may be positioned on opposite sides relative to the chamber, wherein the light beam exits the chamber from the light outlet. The first concave mirror array and the first flat mirror may be coupled together and affixed to the first end of the rectangular housing. The second concave mirror array and the second flat mirror may be coupled together and affixed to the second end of the rectangular housing. The optical path length may be determined based on (i) number of beam spot rows on the first flat mirror or second flat mirror, (ii) number of concave mirrors of the first flat mirror or second flat mirror, and (iii) a mirror-to-mirror distance between the first mirror component and the second mirror component. The number of beam spot rows on the first flat mirror and the second flat mirror may be same, and wherein the number of concave mirrors of the first flat mirror and the second flat mirror may be same. The first mirror component and the second mirror component may define a mirror-to-mirror distance of about 400 mm, and wherein each of the first mirror component and the second mirror component may have a height of about 33 mm and a width of about 111 mm. The first concave mirror array and the second concave mirror array may each comprise 16 concave mirrors. The optical path length may be about 119 meters. The first concave mirror array and the second concave mirror array may each comprise 8 concave mirrors. The optical path length may be about 61 meters.

According to another aspect of the present disclosure, a gas detection system is provided. The gas detection system comprises a compact multi-pass absorption gas cell comprising a rectangular housing defining a chamber for receiving a sample to be analyzed, a light inlet positioned at a first end of the rectangular housing for introducing light beam into the chamber, a light outlet position at a second end of the rectangular housing, a first mirror component positioned at the first end, the first mirror component comprising a first flat mirror and a first concave mirror array, and a second mirror component positioned at the second end, the second mirror component comprising a second flat mirror and a second concave mirror array, wherein the light beam reflects multiple times between the first mirror component and the second mirror component and is refocused by the first concave mirror array and the second concave mirror array to achieve an optical path length for low absorption gas detection. The gas detection system comprises a light emitter configured to emit light beam toward the compact multi-pass absorption gas cell. The gas detection system comprises a light receiver configured to receive light exiting the compact multi-pass absorption gas cell.

According to other aspects of the present disclosure, the gas detection system may include one or more of the following features. The first concave mirror array and the second concave mirror array may each comprise a plurality of concave mirrors. The light beam may reflect between the first mirror component and the second mirror component in a rectangular row-by-row pattern. The light inlet and light outlet may be positioned on opposite sides relative to the chamber, wherein the light beam exits the chamber from the light outlet. The first concave mirror array and the first flat mirror may be coupled together and affixed to the first end of the rectangular housing. The second concave mirror array and the second flat mirror may be coupled together and affixed to the second end of the rectangular housing. The optical path length may be determined based on (i) number of beam spot rows on the first flat mirror or second flat mirror, (ii) number of concave mirrors of the first flat mirror or second flat mirror, and (iii) a mirror-to-mirror distance between the first mirror component and the second mirror component.

The foregoing general description of the illustrative embodiments and the following detailed description thereof are merely exemplary aspects of the teachings of this disclosure and are not restrictive.

### BRIEF DESCRIPTION OF FIGURES

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 illustrates a perspective view of a compact multi-pass absorption gas cell in accordance with at least some example embodiments of the present disclosure.
FIG. 2A illustrates a top view of the compact multi-pass absorption gas cell of FIG. 1 in accordance with at least some example embodiments of the present disclosure.
FIG. 2B illustrates a side view of the compact multi-pass absorption gas cell of FIG. 1 in accordance with at least some example embodiments of the present disclosure.
FIG. 3 illustrates an end view of the compact multi-pass absorption gas cell of FIG. 1 showing light beam refocusing in accordance with at least some example embodiments of the present disclosure.
FIGS. 4A-4D illustrate reflection patterns of a light beam on mirror components in the multi-pass absorption gas cell of FIG. 1 in accordance with at least some example embodiments of the present disclosure.
FIG. 5 illustrates a perspective view of a compact multi-pass absorption gas cell in accordance with at least some example embodiments of the present disclosure.
FIG. 6A illustrates a top view of the compact multi-pass absorption gas cell of FIG. 5 in accordance with at least some example embodiments of the present disclosure.
FIG. 6B illustrates a side view of the compact multi-pass absorption gas cell of FIG. 5 in accordance with at least some example embodiments of the present disclosure.
FIG. 7 illustrates an end view of the compact multi-pass absorption gas cell pf FIG. 5 showing light beam refocusing in accordance with at least some example embodiments of the present disclosure.
FIGS. 8A-8D illustrate reflection patterns of a light beam on mirror components in the multi-pass absorption gas cell of FIG. 5 in accordance with at least some example embodiments of the present disclosure.
FIG. 9 is a block diagram of an example system for gas absorption spectroscopy in accordance with at least some example embodiments of the present disclosure.

### DETAILED DESCRIPTION

Some embodiments of the present disclosure will now be described more fully hereinafter with reference to the accompanying figures, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The term "or" is used herein in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically connected," "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. the term "element" may refer to an article, a device, or an apparatus that may provide one or more functionalities.

### Overview

Various embodiments of the present disclosure relate to compact multi-pass absorption gas cells for use in gas spectroscopy systems. Gas spectroscopy analysis may be utilized to determine one or more components within a sample (e.g., a fluid sample) by illuminating the sample with specific wavelengths of light and monitoring the absorption of light over an optical path length. Each component of a sample may have a unique absorption signature that may be used to identify the presence and concentration of the component within the sample, or in some cases, the absence of a target gas in the sample.

Many target gases are present in low concentrations, such as parts per million or parts per billion. When utilizing spectroscopy to detect and determine concentrations of low concentration gases, long optical path lengths may be utilized. An optical path may be the distance emitted light travels between an illumination source and a receiver, such as a receiving photodetector. Long optical path lengths, such as greater than a meter, may generate sufficient absorption of the light emitted from an illumination source to be measured at a receiving photodetector.

A multi-pass absorption gas detection cell may utilize multiple reflections to achieve long optical paths for low absorption gas detection. Conventional multi-pass absorption gas cells may have limited repetitive reflection count due to non-uniform beam patterns and control spot sizes on mirror surfaces that may be difficult to control. The compactness of conventional multi-pass absorption gas cells may be limited. For example, a multi-pass absorption gas cell, such as a Herriott cell, with two concave mirrors facing each other may reflect input light between the two mirrors multiple times to achieve a total path length. The mirrors may be configured to refocus the light at each reflection to maintain the light beam size. Because the same concave surface re-focuses the beam and redirects the beam in reflection, the beam pattern on the mirror surface may have low surface utilization. Conventional multi-pass absorption gas cells may produce circular light patterns which leave center portions of mirror surfaces unused.

Various embodiments of the present disclosure provide a compact multi-pass absorption gas cell that overcome technical challenges associated with multi-pass absorption gas cells. Various embodiments provide a compact multi-pass absorption gas cell with a high number of reflections and longer total optical path for absorption gas detection, as well as enhanced gas detection sensitivity for lower gas concentration and lower absorption gas detection. A compact multi-pass absorption gas cell with a smaller size relative to conventional gas cells may be provided.

Various embodiments provide a compact multi-pass absorption gas cell that utilizes rectangular row-by-row reflection patterns to minimize mirror size and gas cell size. Various embodiments utilize concave mirror arrays to provide refocused consistent beam size, which may minimize total mirror area. Various embodiments provide a compact multi-pass absorption gas cell having a simplified cell structure that utilizes rectangular light patterns on rectangular mirrors to achieve high compactness. The cell structure may comprise a combination of flat mirrors and concave mirror arrays. Various embodiments may provide a highly compact rectangular multi-pass absorption gas cell that satisfies long optical path gas detection requirements in smaller form factors for various applications, including mobile applications and other portable applications.

### EXAMPLE SYSTEMS AND APPARATUSES OF THE DISCLOSURE

As noted above, various embodiments of the present disclosure provide a compact multi-pass absorption gas cell that overcome technical challenges associated with multi-pass absorption gas cells. Various embodiments provide a compact multi-pass absorption gas cell with a high number of reflections and longer total optical path for absorption gas detection, as well as enhanced gas detection sensitivity for lower gas concentration and lower absorption gas detection.

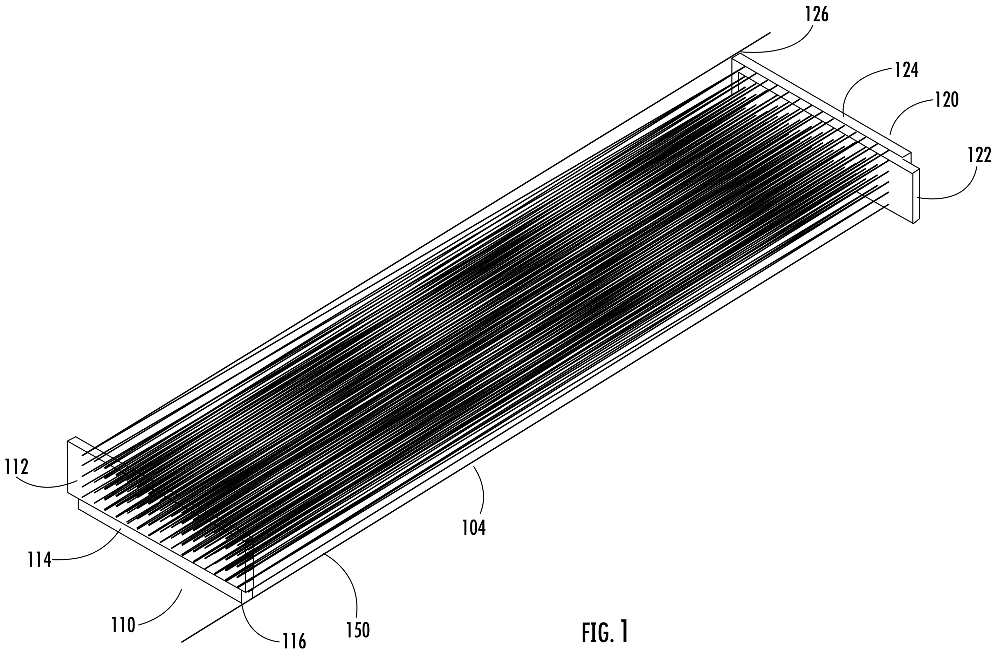

Referring to FIGS. 1-3, a compact multi-pass absorption gas cell 100 may comprise a housing 102 that defines a chamber 104 for receiving a gas to be analyzed. In various embodiments, the housing 102 has a rectangular shape. In this regard, in various embodiments, the compact multi-pass absorption gas cell 100 may comprise a rectangular housing 102. The compact multi-pass absorption gas cell 100 may include an inlet via which the gas to be analyzed is added to the chamber 104 and an outlet via which the gas to be analyzed is removed from the chamber 104. A first mirror component 110 (also referred to herein as first mirror assembly 110) may be affixed (e.g., coupled, secured, or similar terms) to, or otherwise positioned at, a first end of the rectangular housing and a second mirror component 120 (also referred to herein as second mirror assembly 120) may be affixed to, or otherwise positioned at, a second end of the rectangular housing 102. The first mirror component 110 and the second mirror component 120 may be positioned opposite each other to define the chamber 104 between them.

The first mirror component 110 may comprise a first flat mirror 112 and a first concave mirror array 114 (also referred to herein as first turning mirror array 114). In some embodiments, the first concave mirror array 114 may be positioned below the first flat mirror 112 as shown in the illustrated orientation. The first concave mirror array 114 may comprise a plurality of concave mirrors (also referred to herein as concave mirror elements) arranged in a row. The gas cell 100 includes a light inlet 116 (also referred to herein as light inlet portion 116) positioned at a first end relative to the housing 102 or chamber 104 through which light may enter the chamber 104. In some embodiments, the first mirror component 110 may define the light inlet 116.

The second mirror component 120 may comprise a second flat mirror 122 and a second concave mirror array 124 (also referred to herein as second turning mirror array 124). In some embodiments, the second concave mirror array 124 may be positioned above the second flat mirror 122 as shown in the illustrated orientation. The second concave mirror array 124 may comprise a plurality of concave mirrors arranged in a row. The gas cell 100 includes a light outlet 126 (also referred to herein as light outlet portion 126) positioned at a second end relative to the housing 102 or chamber 104 through which light may exit the chamber 104. In some embodiments, the second mirror component 120 may define the light outlet 126. As shown in FIG. 1, the light inlet 116 and light outlet are positioned on opposite sides relative to the chamber 104.

As shown in FIG. 1, a light beam 150 may enter the chamber 104 via the light inlet portion 116. The light beam 150 may reflect back and forth between the first mirror component 110 and the second mirror component 120 within the chamber 104, creating multi-pass optical rays within the chamber 104. After reflecting between the first mirror component 110 and the second mirror component 120 according to a row-by-row reflection pattern, the light beam 150 may exit the chamber 104 through the light outlet portion 126.

In some embodiments, the first concave mirror array 114 and the first flat mirror 112 may be coupled together to form an integrated component. In some examples, the first concave mirror array 114 and the first flat mirror 112 may be CNC machined together as an integrated component. Similarly, the second concave mirror array 124 and the second flat mirror 122 may be coupled together to form an integrated component. In some examples, the second concave mirror array 124 and the second flat mirror 122 may be CNC machined together as an integrated component. In some cases, the first mirror component 110 and the second mirror component 120 may be identical components, which may simplify manufacturing processes.

The housing 102 may be constructed from materials suitable for optical applications, such as aluminum or other metals that provide structural stability. In some embodiments, the housing 102 may be anodized or coated to minimize internal reflections that could interfere with the optical path. The housing 102 may include mounting features for securing the mirror components in precise alignment. The inlet and outlet may be positioned to allow for gas flow through the chamber 104 while minimizing interference with the optical path. In some embodiments, the inlet and outlet may include valves or flow control mechanisms to regulate gas introduction and removal from the chamber 104.

The chamber 104 may have internal dimensions that are optimized for the specific optical path requirements and gas volume needed for effective absorption measurements. The chamber 104 may be sealed to prevent gas leakage and to maintain controlled atmospheric conditions during analysis. The rectangular cross-section of the chamber 104 may complement the rectangular reflection pattern of the light beam 150, providing efficient use of the internal volume while accommodating the systematic arrangement of reflection points on the mirror surfaces.

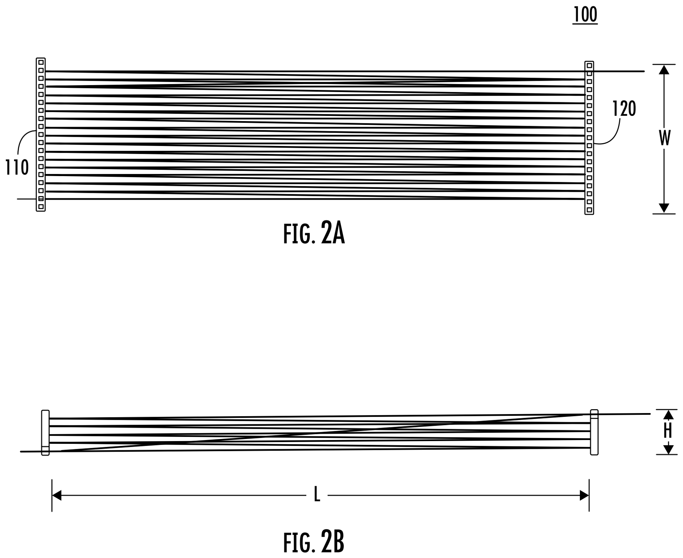

Referring now to FIGS. 2A and 2B, top and side views of the compact multi-pass absorption gas cell 100 illustrating the rectangular housing configuration in accordance with at least some embodiments of the present disclosure is provided. Specifically, FIGS. 2A and 2B show the elongated rectangular form factor of the compact multi-pass absorption gas cell 100. The first mirror component 110 and the second mirror component 120 may be aligned along a longitudinal axis of the housing. The rectangular housing configuration may provide a compact form factor that facilitates efficient use of space while accommodating the optical path requirements for gas absorption analysis.

The first mirror component 110 and the second mirror component 120 may be configured to reflect a light beam in a rectangular row-by-row reflection pattern between the first mirror component 110 and the second mirror component 120. The rectangular configuration of the housing and the arrangement of the mirror components may enable the light beam to follow a systematic reflection pattern that maximizes the utilization of the mirror surfaces. The elongated rectangular form factor may accommodate multiple reflections while maintaining a compact overall size suitable for portable and mobile applications.

The longitudinal axis alignment of the mirror components may ensure that the optical path remains centered within the chamber 104 and that the reflection pattern maintains consistency across the entire length of the gas cell 100. The rectangular form factor may contrast with conventional circular or cylindrical gas cell designs, providing advantages in terms of space utilization and integration into rectangular instrument housings or portable device enclosures. The compact may enable the gas cell 100 to be incorporated into handheld devices or mobile monitoring systems where space constraints are critical considerations.

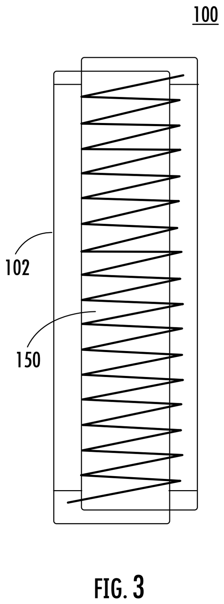

Referring to FIG. 3, an end view of the compact multi-pass absorption gas cell 100 illustrating how the light beam 150 may be refocused between the concave mirror arrays (e.g., first concave mirror array 114 and the second concave mirror array 124) during operation is provided. As shown in FIG. 3, the light beam 150 may traverse between the mirror components while being periodically refocused between the first concave mirror array 114 and the second concave mirror array 124 to maintain consistent beam characteristics throughout the optical path. For example, the first concave mirror array 114 and the second concave mirror array 124 may be configured and positioned to refocus the light beam 150 to, for example, maintain minimum beam divergence during multiple light reflections within the chamber 104.

As described above, the concave mirror arrays may be positioned at opposing ends of the chamber 104, with the first concave mirror array 114 positioned at a lower portion and the second concave mirror array 124 positioned at an upper portion as shown in the illustrated configuration. In some embodiments, the positions of the concave mirror arrays may be reversed. For example, in some embodiments, the concave mirror arrays may be positioned at opposing ends of the chamber 104, with the first concave mirror array 114 positioned at an upper portion and the second concave mirror array 124 positioned at a lower portion. The refocusing action of the concave mirror arrays may maintain the light beam size and minimize beam divergence throughout the multiple reflections within the chamber 104.

In some embodiments, the concave mirrors of the first concave mirror array 114 and the second concave mirror array 124 may have a radius of approximately 1800mm. The concave mirror arrays may be configured to provide consistent refocusing of the light beam 150 as the light beam 150 reflects between the mirror components. The radius of approximately 1800mm may provide appropriate focusing characteristics for maintaining beam quality during extended optical paths. The specific radius value may be selected based on the desired focal length and the physical dimensions of the chamber 104, ensuring that the light beam 150 maintains optimal convergence characteristics throughout the multi-pass system.

The light beam 150 may have an input beam 1/e² radius of 1mm focused at 1800mm from the concave mirror. The input beam parameters may be selected to optimize the refocusing behavior of the concave mirror arrays and maintain consistent beam size throughout the multiple reflections. The focusing distance of 1800mm may correspond to the radius of the concave mirrors, providing appropriate beam convergence and divergence characteristics for the multi-pass optical system. The 1/e² radius specification may define the beam waist where the intensity drops to 1/e² (approximately 13.5%) of the peak intensity, which may be a standard measure for characterizing Gaussian beam profiles in optical systems.

The concave mirror arrays may be designed to operate in a confocal or near-confocal configuration, where the focal points of opposing mirrors may be positioned to optimize beam stability throughout the extended optical path. The confocal arrangement may minimize beam walk-off and maintain consistent spot sizes across all reflection points. Each individual concave mirror within the arrays may be precisely aligned to ensure that the refocusing action occurs at predetermined intervals along the optical path, preventing cumulative beam degradation that could occur in conventional multi-pass systems.

The refocusing mechanism may utilize the principle of periodic focusing, where the light beam 150 may be allowed to diverge slightly between refocusing events while being brought back to a controlled beam waist at each concave mirror interaction. This approach may balance the need for beam control with the practical constraints of mirror spacing and chamber dimensions. The periodic refocusing may occur at intervals determined by the spacing between concave mirror elements within each array, with the interval selected to prevent excessive beam expansion while maintaining efficient use of the available mirror surface area.

As described above, FIG. 3 illustrates how the light beam 150 may be systematically refocused within the chamber 104 as the light beam 150 traverses between the first mirror component 110 and the second mirror component 120 and refocused by the first concave mirror array 114 and the second concave mirror array 124. The refocusing mechanism may prevent excessive beam expansion that could otherwise reduce the effectiveness of the multi-pass absorption system. The concave mirror arrays 114 of the first mirror component 110 and the concave mirror array 124 of the second mirror component 120 may work together to maintain the light beam 150 within acceptable size parameters throughout the extended optical path length.

The beam refocusing may provide several technical advantages over conventional multi-pass gas cells. The systematic refocusing may enable higher reflection counts while maintaining beam quality, as the periodic correction of beam divergence may prevent the gradual degradation that typically limits the number of useful reflections in conventional systems. The refocusing action may also enable more precise control over beam positioning on the mirror surfaces, facilitating the rectangular row-by-row reflection pattern that maximizes mirror surface utilization. Additionally, the maintained beam size may ensure consistent interaction volume with the gas sample throughout the extended optical path, providing uniform sensitivity across all portions of the multi-pass system.

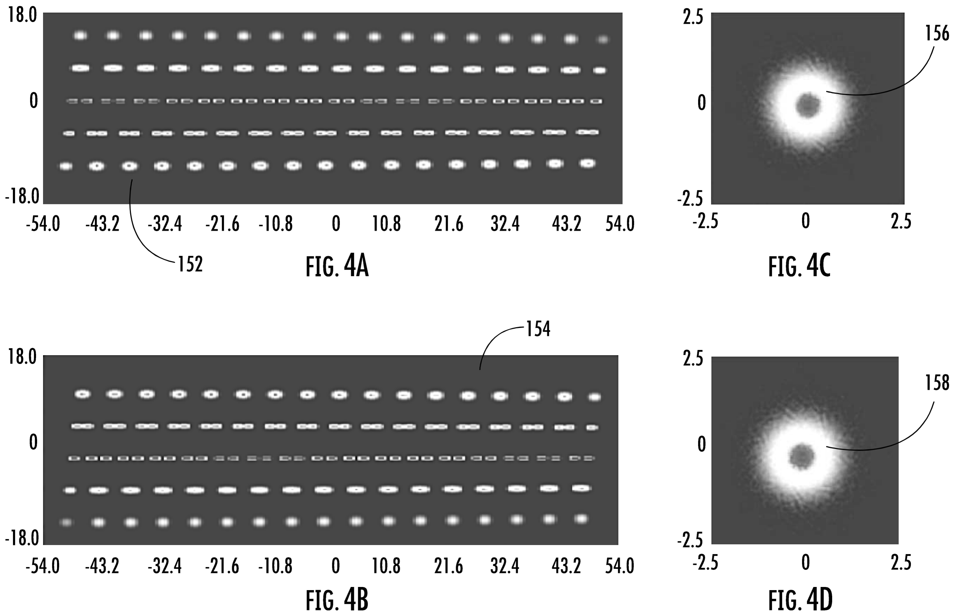

Referring to FIGS. 4A and 4B, reflection patterns of a light beam on the first mirror component 110 and the second mirror component 120 is illustrated in accordance with at least some embodiments of the present disclosure. Specifically, FIGS. 4A and 4B illustrate row-by-row pattern reflection of the light beam 150 on surfaces of the first mirror component 110 and the second mirror component 120 to create an extended optical length in accordance with at least some example embodiments of the present disclosure.

FIG. 4A illustrates row-by-row light spot pattern 152 of the light beam 150 on the first mirror component 210 and FIG. 4B illustrates row-by-row light spot pattern 154 of the light beam 150 on the second mirror component 120. As shown in FIGS. 4A and 4B, multiple light spots may be arranged in row across the surfaces of the mirror components in a rectangular pattern, maximizing utilization of the available mirror surface area. For example, as shown in FIGS. 4A and 4B, the light spots may be organized in horizontal rows across the mirror surfaces, with each row comprising multiple reflection points, wherein the rectangular row-by-row pattern may fully utilize the rectangular mirror surfaces of the first mirror component 110 and the second mirror component 120.

The systematic arrangement of the light spots reflect the folding of the multi-pass optical rays (e.g., defined by the multiple reflections of the light beam 150 between the mirror components) within the compact chamber 104 to achieve extended path lengths while maintaining a compact form factor. The light beam 150 may be reflected between the first flat mirror 112 and the second flat mirror 122 a predetermined number of times, such as 2m times, where m may represent the number of beam spot rows on a given flat mirror (e.g., first flat mirror 112 or second flat mirror 122). After these reflections, the light beam may be refocused with the first and second concave mirror arrays 114, 124, followed by additional reflections between the first and second flat mirrors 112, 122. The refocusing process may occur 2n times, where n may represent the number of concave mirror elements of a given concave mirror array (e.g., number of concave mirrors in the respective first concave mirror array 114 or second concave mirror array 124), followed by additional 2m times reflection between the first flat mirror 112 and the second flat mirror 122. In various embodiments, the number of concave mirrors in the first concave mirror array 114 and the number of concave mirrors in the second concave mirror array 124 are the same.

The row-by-row reflection pattern may enable the compact multi-pass absorption gas cell 100 to achieve a high number of reflections within a limited space. The rectangular arrangement of light spots may contrast with conventional circular patterns that may leave portions of mirror surfaces unused. The row-by-row pattern may provide more efficient use of the mirror surface area, contributing to the compact design of the gas cell while maintaining extended optical path lengths for gas absorption analysis. Additionally, the rectangular pattern may be more compatible with rectangular housing geometries, enabling more efficient packaging of the overall gas cell system.

The spacing between adjacent light spots within each row may be determined by the beam size and the optical design parameters to prevent overlap or interference between adjacent reflection points. Each light spot may represent a discrete reflection event where the light beam interacts with the mirror surface before being redirected to the next reflection point in the sequence. The systematic spacing may ensure that each reflection point receives the full intensity of the light beam without interference from neighboring reflections, thereby maintaining signal quality throughout the extended optical path. The progression of light spots from one row to the next may be controlled by the interaction between the flat mirrors and the concave mirror arrays. When the light beam reaches the end of a row on one mirror component, the corresponding concave mirror array may redirect the light beam 150 to begin a new row on the opposite mirror component. This row-to-row transition may be precisely controlled to ensure that the light beam 150 follows a predictable path without missing reflection points or creating unwanted beam overlap.

The light spot size at each reflection point may be maintained within predetermined tolerances through the refocusing action of the concave mirror arrays. Consistent spot size may enable uniform gas interaction volume at each reflection point, ensuring that the absorption measurements remain consistent throughout the extended optical path. The spot size control may also prevent beam clipping at the mirror edges, which could otherwise introduce optical losses and reduce the overall system efficiency.

FIG. 4C illustrates input characteristics 156 of the light beam 150 before or as the light beam enters the compact multi-pass absorption gas cell 100 and FIG. 4D illustrates output characteristics 158 of the light beam after the light beam exits the compact multi-pass absorption gas cell 100 in accordance with at least some example embodiments of the present disclosure. The input characteristics may reflect the initial beam parameters such as beam diameter, divergence angle, and intensity profile. The input beam positioning may be precisely controlled to ensure that the light beam enters the chamber 104 at the correct location to initiate the systematic row-by-row progression across the mirror surfaces. The output characteristics may reflect the beam parameters after multiple reflections and after the beam exits the chamber 104. The light beam may maintain sufficient beam quality for effective detection and/or absorption measurement. For example, the output beam characteristics may be compared with the input characteristics to determine the absorption signature of the gas sample, with any changes in intensity or spectral content attributable to gas absorption within the chamber 104. The reflection pattern may ensure that the light beam follows a predictable path through the chamber 104, enabling reliable gas absorption measurements. The beam path predictability may facilitate accurate gas concentration measurements.

In some embodiments, the compact multi-pass absorption gas cell 100 achieves multi-pass optical rays folded in 9 segments in one plane with 33 folding planes by using the first and second concave mirror arrays 114, 124. The folding of multi-pass optical rays in 9 segments within one plane may be achieved through the systematic reflection pattern between the first and second mirror components 110, 120. The 33 folding planes may be created by the first and mirror components, which may redirect the light beam through multiple vertical and horizontal levels within the chamber 104. The concave mirror arrays may refocus the beam to maintain minimum beam divergence throughout the extended optical path, ensuring consistent beam quality across all 33 folding planes and 9 segments per plane. In some embodiments, the 9 segments per plane may correspond to the progression of the light beam across the width of the first and second flat mirrors 112, 122 before redirection to the next folding plane. In some embodiments, the 9 segments per plane may correspond to the progression of the light beam across the height of the first and second flat mirrors 112, 122 before redirection to the next folding plane.

The dimensions of the mirror components may be determined based on the maximum beam size and the number of mirror elements. The width W of the mirror components may be calculated using the equation W = (n + 2.5) × a, where a may represent the maximum beam size. The height H of the mirror components may be determined by the equation H = (m + 1.5) × a, where a may represent the maximum beam size. These dimensional relationships may ensure that the mirror components provide adequate surface area to accommodate the rectangular reflection pattern while maintaining compact overall dimensions.

The width and height calculations may incorporate safety margins. For example, the width calculation W = (n + 2.5) × a may incorporate a safety margin of 2.5 beam diameters to account for beam positioning tolerances and potential beam walk-off during operation. This margin may ensure that the light beam remains within the active mirror surface area even under conditions of mechanical vibration or thermal expansion of the housing components. The height calculation H = (m + 1.5) × a may provide adequate clearance for the vertical arrangement of beam spot rows while maintaining compact form/configuration.

In some examples, the maximum beam size parameter 'a' may range from 1mm to 3mm depending on the specific application requirements and the characteristics of the light source. Smaller beam sizes may enable higher reflection densities and more compact mirror dimensions, while larger beam sizes may provide improved signal-to-noise ratios for gas absorption measurements. The selection of beam size may involve trade-offs between compactness, optical efficiency, laser intensity damage level and measurement sensitivity.

The total active volume V of the gas cell 100 may be calculated using the formula V = W × H × L = (m + 1.5) × (n + 2.5) × a² × L. This volume calculation may demonstrate how the rectangular row-by-row ray pattern may fully utilize the rectangular mirror surfaces and enable a minimum volume gas cell in a rectangular form factor. The configuration may provide a compact solution that achieves long optical path requirements while maintaining a small physical footprint suitable for mobile and portable applications.

The optical path length may be calculated using the formula: optical path = (2m + 1) × (2n + 1) × L, where m represents the number of beam spot rows on a single flat mirror (e.g., first flat mirror 112 or second flat mirror 122), n represents the number of concave mirror elements of a single concave mirror array (e.g., first concave mirror array 114 or second concave mirror array 124), and L represents the mirror-to-mirror distance (e.g., distance between the first mirror component 110 and second mirror component 120). The optical path length calculation demonstrates the mathematical relationship between the physical dimensions of the gas cell and the achievable optical path extension. The formula/equation optical path = (2m + 1) × (2n + 1) × L provides a systematic approach for determining the total path length based on the number of reflection segments and folding planes. The factor (2m + 1) may account for the complete traversal of beam spot rows on each flat mirror, including both forward and return paths plus the initial entry. The factor (2n + 1) may represent the total number of interactions with the concave mirror array elements, encompassing all folding planes plus the initial beam entry.

In some embodiments, the compact multi-pass absorption gas cell 100 may have configuration that achieves a total optical path length of approximately 118.8 meters within a compact volume of 400mm × 111mm × 33mm. For example, the compact multi-pass absorption gas cell 100 may have a mirror-to-mirror distance L of about 400m, a mirror component height (e.g., height of a single mirror component) of about 33mm, and a mirror component width (e.g. width of a single mirror component) of about 111mm, 16 concave mirror elements in each concave mirror array (e.g., n = 16), and where m = 4 (e.g. 4 beam spot rows on a single flat mirror), resulting in the total optical path length of approximately 119 (9 x 33 x 0.4) meters within a compact volume of 400mm × 111mm × 33mm.

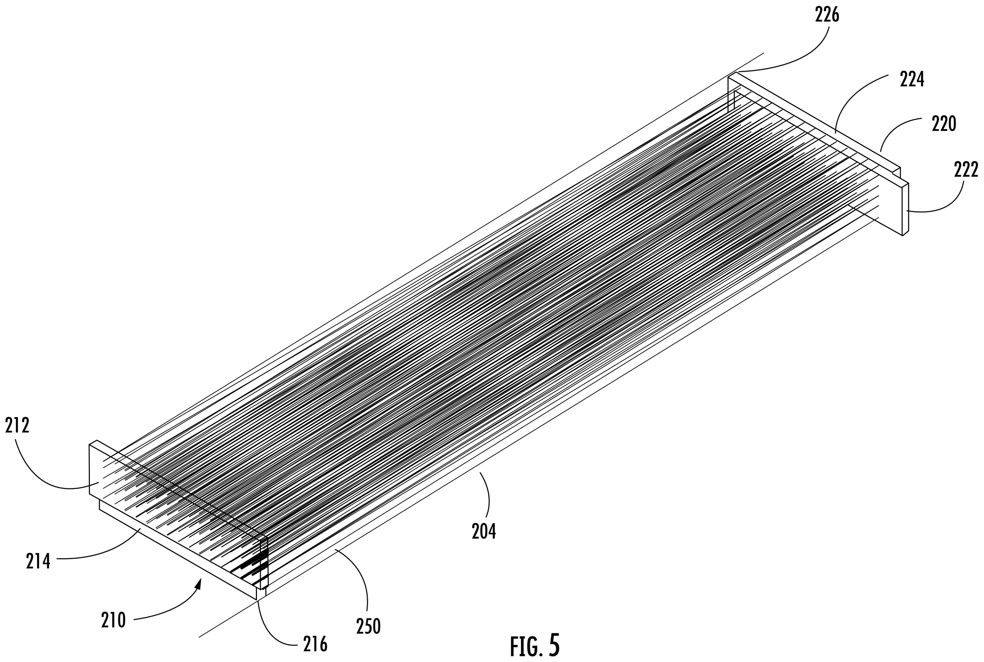

Referring to FIGS. 5-8D, an example compact multi-pass absorption gas cell 200 is provided in accordance with at least some embodiments of the present disclosure. The example compact multi-pass absorption gas cell 200 may implement or otherwise represent a single turning configuration. The single turning configuration may utilize or create a column-by-column, row-by-row pattern on the mirror surfaces. The single turning configuration may utilize fewer folding planes relative to the example higher path length configuration described with reference to the compact multi-pass absorption gas cell 100. The column-by-column, row-by-row pattern may provide efficient utilization of the rectangular mirror surfaces while accommodating the reduced complexity of the single turning arrangement. The compact multi-pass absorption gas cell 200 may comprise similar components as the compact multi-pass absorption gas cell 100

The compact multi-pass absorption gas cell 200 may comprise a housing 202 that defines a chamber 204 for receiving a gas to be analyzed. In various embodiments, the housing 202 has a rectangular shape. In this regard, in various embodiments, the compact multi-pass absorption gas cell 200 may comprise a rectangular housing 202. The compact multi-pass absorption gas cell 200 may include an inlet via which the gas to be analyzed is added to the chamber 204 and an outlet via which the gas to be analyzed is removed from the chamber 204. A first mirror component 210 (also referred to herein as first mirror assembly 210) may be affixed to, or otherwise positioned at, a first end of the rectangular housing, and a second mirror component 220 (also referred to herein as second mirror assembly 220) may be affixed to, or otherwise positioned at, a second end of the rectangular housing 202. The first mirror component 210 and the second mirror component 220 may be positioned opposite each other to define the chamber 204 between them. In this regard, the housing

The first mirror component 210 may comprise a first flat mirror 212 and a first concave mirror array 214 (also referred to herein as first turning mirror array 214). In some embodiments, the first concave mirror array 214 may be positioned below the first flat mirror 212 as shown in the illustrated orientation. The first concave mirror array 214 may comprise a plurality of concave mirrors arranged in a row. The gas cell 200 includes a light inlet 216 (also referred to herein as light inlet portion 216) positioned at a first end relative to the housing 202 or chamber 204 through which light may enter the chamber 204. In some embodiments, the first mirror component 210 may define the light inlet 216.

The second mirror component 220 may comprise a second flat mirror 222 and a second concave mirror array 224 (also referred to herein as second turning mirror array 224). In some embodiments, the second concave mirror array 224 may be positioned above the second flat mirror 222 as shown in the illustrated orientation. The second concave mirror array 224 may comprise a plurality of concave mirrors arranged in a row. The gas cell 200 includes a light outlet 226 (also referred to herein as light outlet portion 226) positioned at a second end relative to the housing 202 or chamber 204 through which light may exit the chamber 204. In some embodiments, the second mirror component 220 may define the light outlet 226.

As shown in FIG. 5, a light beam 250 may enter the chamber 204 via the light inlet portion 216 of the first mirror component 210. The light beam 250 may reflect back and forth between the first mirror component 210 and the second mirror component 220 within the chamber 204, creating multi-pass optical rays with the chamber 204. After reflecting between the first mirror component 210 and the second mirror component 220 according to a column-by-column, row-by-row reflection pattern, the light beam 250 may exit the chamber 204 through the light outlet portion 226.

In some embodiments, the first concave mirror array 214 and the first flat mirror 212 may be coupled together to form an integrated component. In some examples, the first concave mirror array 214 and the first flat mirror 212 may be CNC machined together as an integrated component. Similarly, the second concave mirror array 224 and the second flat mirror 222 may be coupled together to form an integrated component. In some examples, the second concave mirror array 224 and the second flat mirror 222 may be CNC machined together as an integrated component.

The housing 202 may be constructed from materials suitable for optical applications, such as aluminum or other metals that provide structural stability. In some embodiments, the housing 202 may be anodized or coated to minimize internal reflections that could interfere with the optical path. The housing 202 may include mounting features for securing the mirror components in precise alignment. The inlet and outlet may be positioned to allow for gas flow through the chamber 204 while minimizing interference with the optical path. In some embodiments, the inlet and outlet may include valves or flow control mechanisms to regulate gas introduction and removal from the chamber 204.

The chamber 204 may have internal dimensions that are optimized for the specific optical path requirements and gas volume needed for effective absorption measurements. The chamber 204 may be sealed to prevent gas leakage and to maintain controlled atmospheric conditions during analysis. The rectangular cross-section of the chamber 204 may complement the rectangular reflection pattern of the light beam 250, providing efficient use of the internal volume while accommodating the systematic arrangement of reflection points on the mirror surfaces.

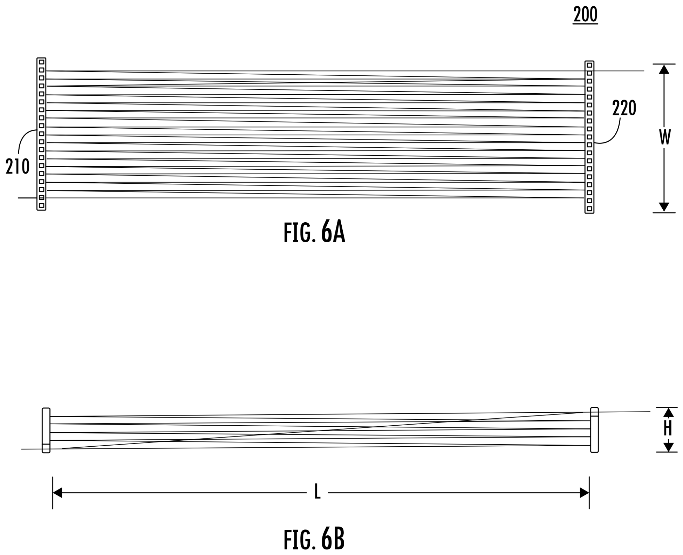

Referring now to FIGS. 6A and 6B, top and side views of the compact multi-pass absorption gas cell 200. illustrating the rectangular housing configuration in accordance with at least some embodiments of the present disclosure is provided. Specifically, FIGS. 6A and 6B show the elongated rectangular form factor of the compact multi-pass absorption gas cell 200. The first mirror component 210 and the second mirror component 220 may be aligned along a longitudinal axis of the housing. The rectangular housing configuration may provide a compact form factor that facilitates efficient use of space while accommodating the optical path requirements for gas absorption analysis.

The first mirror component 210 and the second mirror component 220 may be configured to reflect a light beam in a column-by-column, row-by-row reflection pattern between the first mirror component 110 and the second mirror component 220. The rectangular configuration of the housing 202 and the arrangement of the mirror components may enable the light beam 250 to follow a systematic reflection pattern that maximizes the utilization of the mirror surfaces. The elongated rectangular form factor may accommodate multiple reflections while maintaining a compact overall size suitable for portable and mobile applications.

The longitudinal axis alignment of the mirror components may ensure that the optical path remains centered within the chamber 204 and that the reflection pattern maintains consistency across the entire length of the gas cell 200. The compact form/configuration may enable the gas cell 200 to be incorporated into handheld devices or mobile monitoring systems where space constraints are critical considerations.

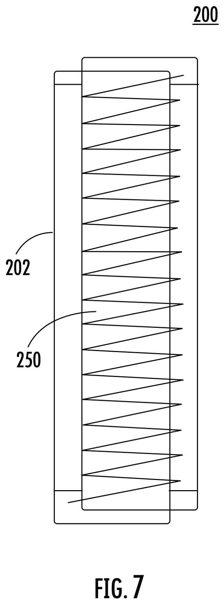

Referring to FIG. 7, an end view of the compact multi-pass absorption gas cell 200 illustrating how the light beam 250 may be refocused between the concave mirror arrays (e.g., first concave mirror array 214 and the second concave mirror array 224) during operation is provided. As shown in FIG. 3, the light beam 250 may traverse between the mirror components while being periodically refocused between the first concave mirror array 214 and the second concave mirror array 224 to maintain consistent beam characteristics throughout the optical path. For example, the first concave mirror array 214 and the second concave mirror array 224 may be configured and positioned to refocus the light beam 250 to, for example, maintain minimum beam divergence during multiple light reflections within the chamber 204.

As described above, the concave mirror arrays may be positioned at opposing ends of the chamber 104, with the first concave mirror array 214 positioned at a lower portion and the second concave mirror array 224 positioned at an upper portion as shown in the illustrated configuration. In some embodiments, the positions of the concave mirror arrays may be reversed. For example, in some embodiments, the concave mirror arrays may be positioned at opposing ends of the chamber 204, with the first concave mirror array 214 positioned at an upper portion and the second concave mirror array 224 positioned at a lower portion. The refocusing action of the concave mirror arrays may maintain the light beam size and minimize beam divergence throughout the multiple reflections within the chamber 204.

In some embodiments, the concave mirrors of the first concave mirror array 214 and the second concave mirror array 224 may have a radius of approximately 1800mm. The concave mirror arrays may be configured to provide consistent refocusing of the light beam 250 as the light beam 250 reflects between the mirror components. The radius of approximately 1800mm may provide appropriate focusing characteristics for maintaining beam quality during extended optical paths. The specific radius value may be selected based on the desired focal length and the physical dimensions of the chamber 204, ensuring that the light beam 250 maintains optimal convergence characteristics throughout the multi-pass system.

The light beam 250 may have an input beam 1/e² radius of 1mm focused at 1800mm from the concave mirror. The input beam parameters may be selected to optimize the refocusing behavior of the concave mirror arrays and maintain consistent beam size throughout the multiple reflections. The focusing distance of 1800mm may correspond to the radius of the concave mirrors, providing appropriate beam convergence and divergence characteristics for the multi-pass optical system.

The concave mirror arrays 214, 224 may be designed to operate in a confocal or near-confocal configuration, where the focal points of opposing mirrors may be positioned to optimize beam stability throughout the extended optical path. The confocal arrangement may minimize beam walk-off and maintain consistent spot sizes across all reflection points. Each individual concave mirror within the arrays may be precisely aligned to ensure that the refocusing action occurs at predetermined intervals along the optical path, preventing cumulative beam degradation that could occur in conventional multi-pass systems. The refocusing mechanism may utilize the principle of periodic focusing, where the light beam 250 may be allowed to diverge slightly between refocusing events while being brought back to a controlled beam waist at each concave mirror interaction. The periodic refocusing may occur at intervals determined by the spacing between concave mirror elements within each concave mirror array, with the interval selected to prevent excessive beam expansion while maintaining efficient use of the available mirror surface area.

As shown in FIG. 7, the light beam 250 may be systematically refocused as the light beam 250 traverses between the first concave mirror array 214 and the second concave mirror array 224. The refocusing mechanism may prevent excessive beam expansion that could otherwise reduce the effectiveness of the multi-pass absorption system. The concave mirror array 214 of the first mirror component 210 and the concave mirror array 224 of the second mirror component 220 may work together to maintain the light beam 250 within acceptable size parameters throughout the extended optical path length.

As described above, the beam refocusing technique may provide several technical advantages over conventional multi-pass gas cells. The systematic refocusing may enable higher reflection counts while maintaining beam quality, as the periodic correction of beam divergence may prevent the gradual degradation that typically limits the number of useful reflections in conventional systems. The refocusing action may also enable more precise control over beam positioning on the mirror surfaces, facilitating the column-by-column, row-by-row reflection pattern that maximizes mirror surface utilization. Additionally, the consistent beam size may ensure consistent interaction volume with the gas sample throughout the extended optical path, providing uniform sensitivity across all portions of the multi-pass system.

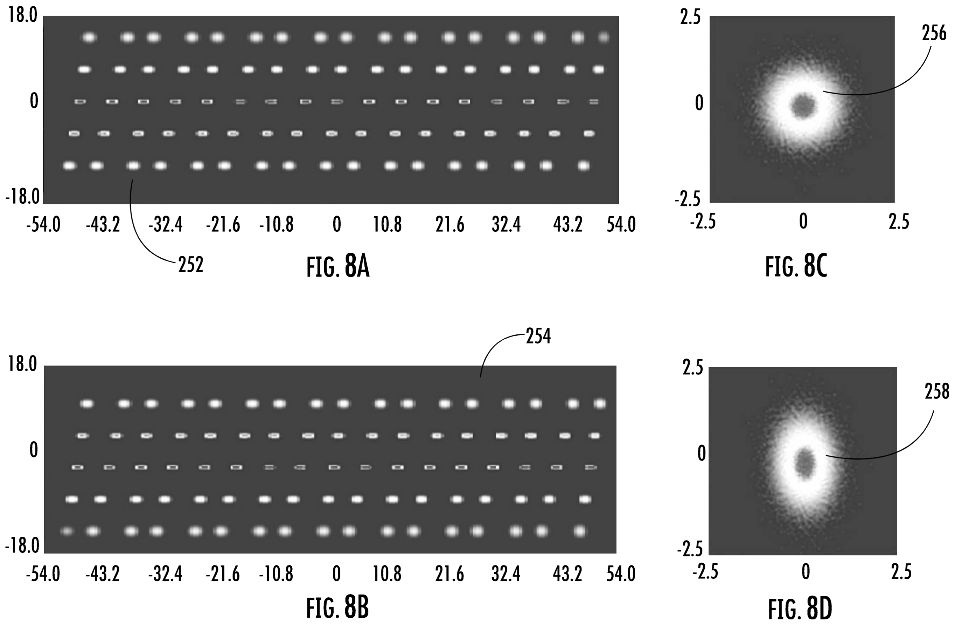

Referring to FIGS. 8A-8B, the reflection pattern of the light beam 250 on the first mirror component 210 and the second mirror component 220 is illustrated in accordance with at least some embodiments of the present disclosure. Specifically, FIG. 8A illustrates column-by-column, row-by-row patterns 252 of the light beam 250 on the first mirror component 210 and FIG. 8B illustrates column-by-column, row-by-row pattern 254 of the light beam 250 on the second mirror component 220.

The column-by-column, row-by-row reflection pattern may provide a grid pattern (e.g., perfect grid pattern) of light spots on the mirror surfaces, which may minimize interference noise. Similar to the example compact multi-pass absorption gas cell 100, the light beam 250 may be reflected between the first flat mirror 212 and the second flat mirror 222 a predetermined number of times, such as 2m times, where m may represent the number of beam spot rows on a given flat mirror (e.g., first flat mirror 212 or second flat mirror 222). After these reflections, the light beam 250 may be refocused with the first and second concave mirror arrays 214, 224, followed by additional reflections between the first and second flat mirrors 212, 222. The refocusing process may occur n times, where n may represent the number of concave mirror elements of a given concave mirror array (e.g., first concave mirror array 214 or second concave mirror array 224), followed by additional 2m times reflection between the first flat mirror 212 and the second flat mirror 222.

The reflection pattern may enable the compact multi-pass absorption gas cell 200 to achieve a high number of reflections within a limited space. The systematic arrangement of the column-by-column, row-by-row pattern may ensure that each reflection point may be positioned to avoid interference with adjacent reflection points, contributing to improved signal quality for gas absorption measurements.

The spacing between adjacent light spots within each row may be determined by the beam size and the optical design parameters to prevent overlap or interference between adjacent reflection points.. The systematic spacing may ensure that each reflection point receives the full intensity of the light beam without interference from neighboring reflections, thereby maintaining signal quality throughout the extended optical path. The progression of light spots from one row to the next may be controlled by the interaction between the flat mirrors and the concave mirror arrays. When the light beam reaches the end of a row on one mirror component, the corresponding concave mirror array may redirect the beam to begin a new row on the opposite mirror component. This row-to-row transition may be precisely controlled to ensure that the light beam follows a predictable path without missing reflection points or creating unwanted beam overlap. The light spot size at each reflection point may be maintained within predetermined tolerances through the refocusing action of the concave mirror arrays.

FIG. 8C illustrates input characteristics 256 of the light beam 250 as the light beam enters the compact multi-pass absorption gas cell 200 and FIG. 8D illustrates output characteristics 258 of the light beam 250 after the light beam exits the compact multi-pass absorption gas cell 100 in accordance with at least some example embodiments of the present disclosure. As shown in FIG. 8C, the light beam 250 may maintain sufficient beam quality for effective detection and/or absorption measurement.

In some embodiments, the compact multi-pass absorption gas cell 200 achieves multi-pass optical rays folded in 9 segments in one plane with 17 folding planes by using the first and second concave mirror arrays 214, 224. The folding of multi-pass optical rays in 9 segments within one plane with 17 folding planes may be achieved through the systematic column-by-column, row-by-row reflection pattern between the first and second mirror components 210, 220. The concave mirror arrays may refocus the beam to maintain minimum beam divergence throughout the extended optical path, ensuring consistent beam quality across the 17 folding planes and 9 segments per plane. In some embodiments, the 9 segments per plane may correspond to the progression of the light beam across the width of the first and second flat mirrors 212, 222 before redirection to the next folding plane. In some embodiments, the 9 segments per plane may correspond to the progression of the light beam across the height of the first and second flat mirrors 212, 222 before redirection to the next folding plane.

The dimensions of the mirror components may be determined based on the maximum beam size and the number of mirror elements as described above with reference to the example compact multi-pass absorption gas cell 100. The total active volume V of the gas cell 200 may be calculated using similar formula as described above with reference to the example compact multi-pass absorption gas cell 100. The optical path length may be calculated using similar optical length formula as described above with reference to the example compact multi-pass absorption gas cell 100.

The optical path length may be calculated using the formula: optical path = (2m + 1) × (n + 1) × L, where m represents the number of beam spot rows on a single flat mirror (e.g., first flat mirror 212 or second flat mirror 222), n represents the number of concave mirror elements of a single concave mirror array (e.g., first concave mirror array 214 or second concave mirror array 224), and L represents the mirror-to-mirror distance (e.g., distance between the first mirror component 210 and second mirror component 220). The optical path length calculation demonstrates the mathematical relationship between the physical dimensions of the gas cell and the achievable optical path extension. The formula/equation optical path = (2m + 1) × (n + 1) × L provides a systematic approach for determining the total path length based on the number of reflection segments and folding planes. The factor (2m + 1) may account for the complete traversal of beam spot rows on each flat mirror, including both forward and return paths.

In some embodiments, the compact multi-pass absorption gas cell 200 may have configuration that achieves a total optical path length of approximately 61.2 meters within a compact volume of 400mm × 111mm × 33mm. For example, the compact multi-pass absorption gas cell 200 may have a mirror-to-mirror distance L of about 400m, a mirror component height (e.g., height of a single mirror component) of about 33mm, and a mirror component width (e.g. width of a single mirror component) of about 111mm, 16 concave mirror elements in each concave mirror array (e.g., n = 16, and where m = 4 (e.g. 4 beam spot rows on a single flat mirror), resulting in the total optical path length of approximately 61.2 (9 x 17 x 0.4) meters within a compact volume of 400mm × 111mm × 33mm.

### Example Multi-Pass Absorption Gas Detection System

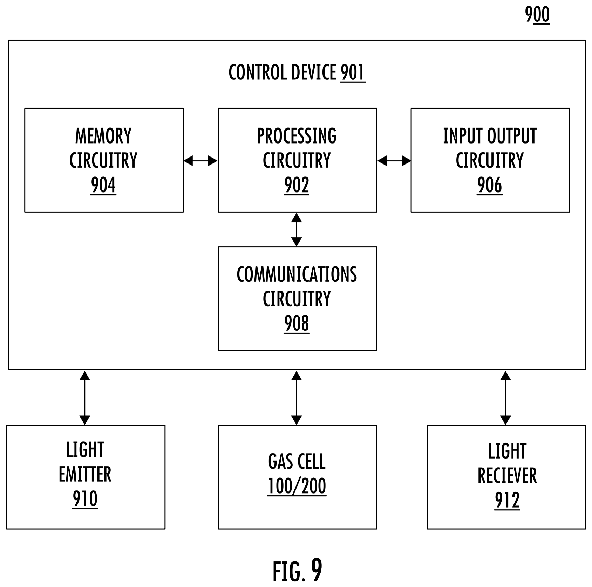

Referring now to FIG. 9, a block diagram of an example multi-pass absorption gas detection system 900 in accordance with example embodiments of the present disclosure is provided. The example system 900 may include a compact multi-pass absorption gas cell such as the example gas cell 100 or gas cell 200, a control device 901, light emitter 910, and/or light receiver 912. The light emitter 910 may be configured to emit light towards and/or into the gas cell 100, as described above. The light receiver 912 may be configured to receive light that exits the gas cell 100, as described above.

The example control device 901 show in FIG. 9 comprises a processor or processing circuitry 902, memory circuitry 904, input/output circuitry 906, and communications circuitry 908. In some embodiments, one or more portions of the control device 901 (e.g., one or more of the components thereof) are configured to execute and perform the operations described herein. In some embodiments, the control device 901 may be configured to control operation of the light emitter 910, the light receiver 912, and/or the gas cell 100. For example, in such some embodiments, the control device 901 may function as a controller within the system 900. In some embodiments,, the control device 901 may be configured to analyze the output from the gas cell 100 or received by the light receiver 912 to determine the presence of one or more target gases.

The control device 901 may be in communication with both the light emitter 910 and the light receiver 912. The control device 901 may control operation of the light emitter 910 to emit light at specified wavelengths and intensities. The control device 901 may receive signals from the light receiver 912 corresponding to the detected light. The processing circuitry 902 may analyze the received signals to determine absorption characteristics of a gas sample, thereby identifying presence and concentration of one or more target gases within the chamber of the gas cell.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitries both leverage use of the same processor(s), memory(ies), circuitry(ies), and/or the like to perform their associated functions such that duplicate hardware is not required for each set of circuitries.

Processing circuitry 902 may be embodied in a number of different ways. In various embodiments, the use of the terms processor or processing circuitry should be understood to include a single core processor, a multi-core processor, multiple processors internal to the example device 901, and/or one or more remote or cloud processor(s) external to the example device 901. In some example embodiments, processing circuitry 902 may include one or more processing devices configured to perform independently. Alternatively, or additionally, processing circuitry 902 may include one or more processor(s) configured in tandem via a bus to enable independent execution of operations, instructions, pipelining, and/or multithreading.

In an example embodiment, the processing circuitry 902 may be configured to execute instructions stored in the memory circuitry 904 or otherwise accessible to the processor. Alternatively, or additionally, the processing circuitry 902 may be configured to execute hard-coded functionality. As such, whether configured by hardware or software methods, or by a combination thereof, processing circuitry 902 may represent an entity (e.g., physically embodied in circuitry) capable of performing operations according to embodiments of the present disclosure while configured accordingly. Alternatively, or additionally, processing circuitry 902 may be embodied as an executor of software instructions, and the instructions may specifically configure the processing circuitry 902 to perform the various algorithms embodied in one or more operations described herein when such instructions are executed. In some embodiments, the processing circuitry 902 includes hardware, software, firmware, and/or a combination thereof that performs one or more operations described herein.

The processing circuitry 902 may implement various signal processing algorithms to extract meaningful absorption data from the received optical signals. The processing circuitry 902 may perform baseline correction to account for variations in light source intensity and detector response characteristics. The processing circuitry 902 may apply digital filtering techniques to reduce noise and improve signal-to-noise ratios in the absorption measurements. The processing circuitry 902 may execute spectral analysis algorithms that identify characteristic absorption peaks corresponding to specific molecular transitions of target gases.

In some embodiments, the processing circuitry 902 (and/or co-processor or any other processing circuitry assisting or otherwise associated with the processor) is/are in communication with the memory circuitry 904 via a bus for passing information among components of the example device 901.

Memory or memory circuitry 904 may be non-transitory and may include, for example, one or more volatile and/or non-volatile memories. In some embodiments, the memory circuitry 904 includes or embodies an electronic storage device (e.g., a computer readable storage medium). In some embodiments, the memory circuitry 904 is configured to store information, data, content, applications, instructions, or the like, for enabling the example device 901 to carry out various operations and/or functions in accordance with example embodiments of the present disclosure.

The memory circuitry 904 may store reference absorption spectra for various target gases, enabling the processing circuitry 902 to perform pattern matching and identification of unknown gas components. The memory circuitry 904 may maintain calibration data that relates measured absorption signals to actual gas concentrations, accounting for factors such as temperature, pressure, and optical path length variations. The memory circuitry 904 may store historical measurement data for trend analysis and long-term monitoring applications. The memory circuitry 904 may include both volatile memory for real-time data processing and non-volatile memory for persistent storage of calibration parameters and reference data.

Input/output circuitry 906 may be included in the example device 901. In some embodiments, input/output circuitry 906 may provide output to the user and/or receive input from a user. The input/output circuitry 906 may be in communication with the processing circuitry 902 to provide such functionality. The input/output circuitry 906 may comprise one or more user interface(s). In some embodiments, a user interface may include a display that comprises the interface(s) rendered as a web user interface, an application user interface, a user device, a backend system, or the like. In some embodiments, the input/output circuitry 906 also includes a keyboard, a mouse, a joystick, a touch screen, touch areas, soft keys a microphone, a speaker, or other input/output mechanisms. The processing circuitry 902 and/or input/output circuitry 906 may be configured to control one or more operations and/or functions of one or more user interface elements through computer program instructions (e.g., software and/or firmware) stored on a memory accessible to the processor (e.g., memory circuitry 904, and/or the like). In some embodiments, the input/output circuitry 906 includes or utilizes a user-facing application to provide input/output functionality to a computing device and/or other display associated with a user. In some embodiments, the input/output circuitry 906 one or more indicator lights or the like for providing a user notification (e.g., an alert or warning).

Communications circuitry 908 may be included in the example device 901. The communications circuitry 908 may include any means such as a device or circuitry embodied in either hardware or a combination of hardware and software that is configured to receive and/or transmit data from/to a network and/or any other device, circuitry, or module in communication with the example device 901. In some embodiments the communications circuitry 908 includes, for example, a network interface for enabling communications with a wired or wireless communications network. Additionally, or alternatively, the communications circuitry 908 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 908 may include circuitry for interacting with an antenna(s) and/or other hardware or software to cause transmission of signals via the antenna(s) and/or to handle receipt of signals received via the antenna(s). In some embodiments, the communications circuitry 908 enables transmission to and/or receipt of data from a user device, one or more sensors, and/or other external computing device(s) in communication with the example device 901.

In some embodiments, two or more of the sets of circuitries 902-908 are combinable. Alternatively, or additionally, one or more of the sets of circuitries 902-908 perform some or all of the operations and/or functionality described herein as being associated with another circuitry. In some embodiments, two or more of the sets of circuitries 902-908 are combined into a single module embodied in hardware, software, firmware, and/or a combination thereof.

The light emitter 910 may be configured to provide broadband illumination covering multiple absorption bands of interest, enabling simultaneous detection of multiple gas species within a single measurement cycle. The light emitter 910 may include wavelength stabilization mechanisms to ensure consistent spectral output over varying environmental conditions such as temperature fluctuations. The light emitter 910 may incorporate intensity modulation capabilities that enable lock-in detection techniques for improved sensitivity and noise rejection. The light emitter 910 may be designed with sufficient optical power to compensate for losses incurred during the extended multi-pass optical path while maintaining safe operation levels.

The light receiver 912 may comprise photodetectors optimized for the specific wavelength ranges of interest for target gas detection. The light receiver 912 may include multiple detector elements arranged to simultaneously monitor different spectral regions, enabling multi-gas detection capabilities within a single compact system. The light receiver 912 may incorporate signal conditioning electronics that amplify and filter the detected optical signals before conversion to digital format for processing by the control device 901. The light receiver 912 may include temperature stabilization or compensation mechanisms to maintain consistent detector response characteristics across varying operating conditions.

A method for gas absorption spectroscopy may comprise introducing a gas sample into the chamber of the gas cell such as the gas cell 100 or gas cell 200. The gas sample may be introduced through an inlet of the compact multi-pass absorption gas cell 100 and may fill the chamber for analysis. The method may further comprise directing the light beam into the chamber 104 through the light inlet.

The method may include reflecting the light beam multiple times between the first mirror component and the second mirror component positioned opposite the first mirror component, wherein the first mirror component comprises a first flat mirror and a first concave mirror array the second mirror component comprises a second flat mirror and a second concave mirror array. The multiple reflections may follow the row-by-row pattern described above, enabling the light beam to traverse an extended optical path within the compact chamber.

The method may further comprise refocusing the light beam via the first concave mirror array and the second concave mirror array to maintain beam size during the multiple reflections. The refocusing action may prevent beam divergence that could otherwise reduce the effectiveness of the gas absorption measurements. The method may include detecting the light beam after the multiple reflections through the light outlet. The light receiver may detect the light beam and convert optical signals into electrical signals for subsequent analysis.

The method may include performing system calibration procedures prior to gas sample analysis to establish baseline optical characteristics and verify proper system operation. The calibration procedures may involve introducing reference gas samples with known concentrations to establish calibration curves relating measured absorption signals to actual gas concentrations. The method may include temperature and pressure compensation procedures that account for environmental variations that could affect gas absorption characteristics or optical system performance.

The method may comprise implementing real-time quality control measures during gas sample analysis to ensure measurement reliability and accuracy. The quality control measures may include monitoring system parameters such as light source stability, detector response consistency, and optical alignment integrity. The method may include automatic error detection and correction procedures that identify and compensate for systematic measurement errors or drift in system performance over time.

The method may further comprise analyzing the detected light beam to determine presence and concentration of one or more target gases in the gas sample. The processing circuitry 902 may compare the detected light characteristics with reference absorption signatures to identify specific gases and quantify their concentrations. Each gas component may have a unique absorption signature that may be used to distinguish different gases within the sample.

The analysis process may involve spectral deconvolution techniques that separate overlapping absorption features from different gas components, enabling accurate quantification of individual gas concentrations in complex gas mixtures. The analysis may include statistical processing methods that provide confidence intervals and uncertainty estimates for measured concentration values. The analysis may incorporate machine learning algorithms that improve identification accuracy and reduce false positive detection rates through pattern recognition and adaptive learning from historical measurement data.

For example, the compact multi-pass absorption gas cell may be configured for detecting methane and carbon dioxide gases, which may be common targets for environmental monitoring and industrial applications. The compact multi-pass absorption gas cell may be particularly suitable for detecting gases present in low concentrations such as parts per million or parts per billion. The extended optical path length achieved through the multiple reflections may provide sufficient absorption signal strength to detect these low concentration gases with acceptable sensitivity.

The system may be configured for detecting other target gases including but not limited to hydrogen sulfide, ammonia, carbon monoxide, nitrogen oxides, sulfur dioxide, and various volatile organic compounds depending on the specific application requirements. The wavelength range and optical configuration may be optimized for specific gas detection applications, with the light emitter 910 and light receiver 912 selected to provide optimal sensitivity for the target gas absorption bands. The system may include interchangeable optical filters or detector elements that enable reconfiguration for different target gas species without requiring complete system replacement.

The compact multi-pass absorption gas cell may be configured for mobile and portable applications due to the compact form factor achieved through the rectangular row-by-row reflection pattern and efficient mirror surface utilization. The compact design may enable gas detection capabilities to be deployed in field applications rather than being limited to laboratory environments. The compact multi-pass absorption gas cell 100 may be used in industrial, environmental, healthcare, and pharmaceutical domains where gas detection and monitoring may be required for safety, compliance, or process control purposes.

The mobile and portable configuration may include battery power systems that enable extended operation in remote locations without access to external power sources. The portable design may incorporate ruggedized housing and shock-resistant mounting systems that protect the optical components during transportation and field deployment. The mobile configuration may include GPS positioning capabilities that enable location-tagged measurements for environmental mapping and compliance monitoring applications.

The system may be configured for integration into vehicle-mounted monitoring platforms for mobile emission monitoring, leak detection surveys, or environmental assessment applications. The compact form factor may enable installation in confined spaces such as aircraft, ships, or industrial equipment where space constraints limit the use of conventional gas detection systems. The portable design may facilitate rapid deployment for emergency response applications where immediate gas detection capabilities are required at incident sites or disaster areas.

While the description above provides an example system 900 and example control device 901, it is noted that the scope of the present disclosure is not limited to the description above. In some examples, an example system 900 and/or control device in accordance with the present disclosure may be in other forms. In some examples, an example system 900 and/or control device 901 may comprise one or more additional and/or alternative elements, and/or may be structured differently than that illustrated in FIG. 9.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some examples one or more of the steps depicted may occur substantially simultaneously, or additional steps may be involved. Although specific terms are employed herein, they are used in a generic and descriptive sense only and not for purposes of limitation.

## Claims

1. A compact multi-pass absorption gas cell comprising:
a rectangular housing defining a chamber for receiving a sample to be analyzed;
a light inlet positioned at a first end of the rectangular housing for introducing light beam into the chamber;
a light outlet position at a second end of the rectangular housing;
a first mirror component positioned at the first end, the first mirror component comprising a first flat mirror and a first concave mirror array; and
a second mirror component positioned at the second end, the second mirror component comprising a second flat mirror and a second concave mirror array, wherein the light beam reflects multiple times between the first mirror component and the second mirror component and is refocused by the first concave mirror array and the second concave mirror array to achieve an optical path length for low absorption gas detection.

2. The compact multi-pass absorption gas cell of claim 1, wherein the first concave mirror array and the second concave mirror array each comprise a plurality of concave mirrors.

3. The compact multi-pass absorption gas cell of claim 1, wherein the light beam reflects between the first mirror component and the second mirror component in a rectangular row-by-row pattern.

4. The compact multi-pass absorption gas cell of claim 1, wherein the light inlet and light outlet are positioned on opposite sides relative to the chamber, wherein the light beam exits the chamber from the light outlet.

5. The compact multi-pass absorption gas cell of claim 1, wherein the first concave mirror array and the first flat mirror are coupled together and affixed to the first end of the rectangular housing.

6. The compact multi-pass absorption gas cell of claim 1, wherein the second concave mirror array and the second flat mirror are coupled together and affixed to the second end of the rectangular housing.

7. The compact multi-pass absorption gas cell of claim 1, wherein the optical path length is determined based on (i) number of beam spot rows on the first flat mirror or second flat mirror, (ii) number of concave mirrors of the first flat mirror or second flat mirror, and (iii) a mirror-to-mirror distance between the first mirror component and the second mirror component.

8. The compact multi-pass absorption gas cell of claim 7, wherein the number of beam spot rows on the first flat mirror and the second flat mirror are same, and wherein the number of concave mirrors of the first flat mirror and the second flat mirror are same.

9. The compact multi-pass absorption gas cell of claim 1, wherein the first mirror component and the second mirror component define a mirror-to-mirror distance of about 400mm, and wherein each of the first mirror component and the second mirror component have a height of about 33mm and a width of about 111mm.

10. The compact multi-pass absorption gas cell of claim 9, wherein the first concave mirror array comprises 16 concave mirrors.

11. The compact multi-pass absorption gas cell of claim 10, wherein the second concave mirror array comprises 16 concave mirrors.

12. The compact multi-pass absorption gas cell of claim 10, wherein the optical path length is about 119 meters.

13. The compact multi-pass absorption gas cell of claim 12, wherein the optical path length is about 61 meters.

14. A gas detection system comprising:
a compact multi-pass absorption gas cell comprising:
a rectangular housing defining a chamber for receiving a sample to be analyzed;
a light inlet positioned at a first end of the rectangular housing for introducing light beam into the chamber;
a light outlet position at a second end of the rectangular housing;
a first mirror component positioned at the first end, the first mirror component comprising a first flat mirror and a first concave mirror array; and
a second mirror component positioned at the second end, the second mirror component comprising a second flat mirror and a second concave mirror array, wherein the light beam reflects multiple times between the first mirror component and the second mirror component and is refocused by the first concave mirror array and the second concave mirror array to achieve an optical path length for low absorption gas detection;
a light emitter configured to emit light beam toward the compact multi-pass absorption gas cell; and
a light receiver configured to receive light exiting the compact multi-pass absorption gas cell.

15. The gas detection system of claim 14, wherein the first concave mirror array and the second concave mirror array each comprise a plurality of concave mirror.
